# EUROPÄISCHE PATENTSCHRIFT

(11) **EP 1 350 490 B1**
(45) Veröffentlichungstag und Bekanntmachung des Hinweises auf die Patenterteilung: **27.12.2006**
(21) Anmeldenummer: 03013559.4
(22) Anmeldetag: 08.11.1995
(51) Int. Cl.: A61F 2/44, A61F 2/46, A61B 17/32

(54) **Vorrichtung zum Einbringen eines Implantats, insbesondere einer Zwischenwirbelprothese**
Device for inserting an implant, in particular an intervertebral prosthesis
Dispositif pour placer un implant, notamment une prothèse intervertébrale

(43) Veröffentlichungstag der Anmeldung: 08.10.2003
(62) Teilanmeldung aus: 95810701.3
(73) Patentinhaber: Zimmer GmbH, 8404 Winterthur (CH)
(72) Erfinder: Husson, Jean-Louis, Prof. Dr., 35000 Rennes (FR); Baumgartner, Walter, Dr.sc.nat., 8623 Wetzikon (CH); Freudiger, Stefan, 3047 Bremgarten (CH)
(74) Vertreter: Manitz, Finsterwald & Partner GbR

(56) Entgegenhaltungen:
- EP-A- 0 621 020
- EP-A- 0 700 671
- FR-A- 2 712 486
- US-A- 3 448 737
- US-A- 5 176 647
- US-A- 5 263 927

## Beschreibung

Die Erfindung betrifft eine Vorrichtung zum Einbringen eines Implants, insbesondere einer Zwischenwirbelprothese bestehend aus einem länglichen, elastischen Körper, gemäß dem Oberbegriff des Anspruchs 1. Eine Prothese dieser Art ist aus EP-A-0 453 393 bekannt. Sie dient zum Ersatz eines beschädigten Bandscheibenkernes (nucleus pulposus). Dabei bleibt der äussere Ring (anulus fibrosus) einer natürlichen Bandscheibe weitgehend erhalten. Die bekannte Prothese besteht aus einem flüssigkeitsdichten Hohlkörper, der mit einem inkompressiblen, fliessfähigen Medium gefüllt ist. Diese Prothese benötigt zu ihrer Implantation ein besonders ausgebildetes Einführungsinstrument, mit dem der Hohlkörper intra-operativ zu Spiralform aufgewickelt und anschliessend gefüllt werden kann. Dies stellt hohe Anforderungen an die Geschicklichkeit des Operateurs einerseits, sowie an die Herstellung des Einführungsinstruments hinsichtlich des Aufwickelmechanismus, sowie hinsichtlich der Füllvorrichtung. Ferner stellen die Dichtheitskriterien des Hohlkörpers in Anbetracht der hohen Belastungen im Wirbelsäulenbereich auf Dauer ein Risiko dar. Nachdem der Körper im Zwischenwirbelbereich aufgerollt und gefüllt worden ist, liegt das freie Ende am anulus fibrosus an, falls das Hilfswerkzeug, welches zum Füllen und Abdichten im Zentrum der Spirale angreift, entfernbar ist ohne Hohlräume zu erzeugen. Nur wenn kein Abwandern von Spiralenteilen in andere Hohlräume erfolgen kann, können die bei Belastung auftretenden, radial gerichteten Kräfte kompensiert werden und die Spirale erhält die gewünschte Stützfunktion.

Aus FR 2 712 486 ist eine Zwischenwirbelprothese in Form eines biegsamen Bandes bekannt, das mit Hilfe eines weiteren Bandes eingebracht wird. US 5,263,927 beschreibt einen Abroller für weiches Füllmaterial.

Aufgabe der Erfindung ist es, eine Vorrichtung der eingangs genannten Art zu schaffen, die eine einfache Handhabung des Implantats erlaubt.

Diese Aufgabe wird gelöst durch die Merkmale des Anspruchs 1.

Ein mit einer derartigen Vorrichtung einbringbares Implantat kann einen länglichen, elastischen Körper umfassen, der formelastisch ist und im kräftefreien Zustand die Form einer Spirale annimmt. Der Querschnitt des Körpers ist vorzugsweise rechteckig, wobei die Kanten auch abgerundet sein können. Es ist auch möglich Körper mit rundlichem Querschnitt zu verwenden, solange bei Belastung eine im wesentlichen formschlüssige, verkantungssichere Lage der Spirale erhalten bleibt. Der Querschnitt des elastischen Körpers muss nicht konstant sein über seine ganze Länge, vielmehr stellt sich heraus, dass die Dicke vorzugsweise nach aussen hin abnehmen kann. Eine derartige Spirale hat den Vorteil, dass sie sich selbst ablegt, beim Einstossen nicht knickt und in einer passenden aufgewickelten Länge abtrennbar ist.

Das Implantat kann als solches die Form einer Zylinderscheibe annehmen, also flach ausgebildet sein, oder aber den Konturen der Wirbelflächen folgend Wölbungen nach aussen und/oder innen aufweisen. Dadurch wird erreicht, dass sich die bei Belastung erzeugten Kräfte gleichmässiger auf die Wirbelflächen verteilen. Der Durchmesser der Spirale ist vorzugsweise so bemessen, dass der Raum innerhalb des anulus fibrosus ausgefüllt wird.

Um die Bandscheibe einzuführen, hat es sich als vorteilhaft erwiesen, Versteifungsmittel in Form eines Kunststoff- oder Metallbandes am freien Ende der Spirale anzubringen, über welches eine Abtrennvorrichtung bis an eine vorgesehene Trennstelle geführt werden kann. Das Band weist im kräftefreien Zustand vorzugsweise keine Krümmung auf. Nach erfolgter Implantation wird das Band zusammen mit dem nicht benötigten Spiralenanteil von der abgelegten Spirale mittels der Abtrennvorrichtung abgetrennt.

Um die Lage des Implantates intra- und post-operativ kontrollieren zu können, empfiehlt sich das Vorsehen von röntgen-positiven Markierungen in Form von Kugeln oder eines Fadens auf bzw. entlang innerhalb des elastischen Körpers.

Das Material, aus dem der elastische Körper hergestellt ist, soll im wesentlichen den elastischen Eigenschaften einer Bandscheibe entsprechen, sodass die auftretenden Kräfte möglichst gleichmässig auf die Wirbelfläche übertragen werden. Als vorteilhaft hat sich die Verwendung von Polyurethan oder Polyurethan-Silicongemischen herausgestellt, welche eine Shore-Härte im Bereich von etwa 80A aufweisen. Verwendet werden können auch Schäume verschiedenster Kunststoffe, sofern sie den elastischen Anforderungen genügen.

In einer besonders bevorzugten Ausführung weist die Spriale ein zentrales Mittelstück auf, um welches sich die weiteren Windungen dicht anlegen. Es entstehen nur kleine Zwischenräume, die sich gegebenenfalls im implantierten Zustand mit Bandscheibenflüssigkeit füllen und die ein Verkanten des elastischen Körpers unter Radialkräften nicht zulassen. Durch ein geeignetes Höhen- zu Breitenverhältnis wird ein Verkanten des elastischen Körpers generell verhindert. Das Mittelstück kann grundsätzlich eine kreiszylindrische Form aufweisen, aber auch Formen wie Kugel oder Ellipsoid haben sich als nützlich erwiesen. Daran schliesst sich vorteilhafter Weise ein kurzer, dünnerer Übergangsteil, über den die Verbindung zur weiteren Spirale herstellt ist. Dies hat den Vorteil, dass die Spirale in dichtester, flächenschlüssiger Packung verbleibt und die grösstmögliche Flächenverteilung unter Abbau von Kraftspitzen erreicht wird. In einer einfacheren Ausgestaltung des Implantates kann das zentrale Mittelstück auch weggelassen werden, da sein Flächenbeitrag im Verhältnis zur Gesamtfläche klein ist.

Die Höhe der Spirale entspricht im wesentlichen dem physiologischen Zwischenwirbelabstand. Aus den elastischen Eigenschaften des verwendeten Materials ergibt sich die bevorzugte Dicke, in der der elastische Körper auszubilden ist. An der Peripherie der Spirale, nach etwa drei bis fünf Windungen, je nach Material und Breite der Windung, kann die Dicke des Körpers abnehmen, da die anliegenden Kräfte primär zentral aufgenommen werden sollen und da andererseits der Körper an entsprechender Stelle abtrennbar sein soll. In einer weiteren Ausgestaltung ist es möglich, die Höhe des Implantates den individuellen Wirbelanständen anzupassen.

Das Abtrennen soll vorzugsweise innerhalb des Hohlraumes erfolgen, wofür die entsprechenden Kräfte mittels eines noch näher zu beschreibenden Instrumentes aufgebracht werden müssen.

Bei einem Verfahren zur Herstellung eines Zwischenwirbelimplantates in Spiralform kann das Implantat im einfachsten Fall z.B. aus einer Werkstoffplatte mit entsprechender Dicke ausgeschnitten werden. Als Schneidmittel sind herkömmliche Sägen, Schneiddrähte oder ähnliche Werkzeuge verwendbar. Es können aber auch Laser Verwendung finden, die sich zur Bearbeitung von Kunststoffen eignen. Insbesondere Laser, die ein athermisches Schneiden ermöglichen, wie z.B. Excimer-Laser, sind dabei von Vorteil, da sie die Entstehung von toxischen Nebenprodukten weitgehend verhindern. Als besonders bevorzugtes Verfahren erweist sich ein Spritzgussverfahren, wobei die schneckenförmige Gussform vorzugsweise im Spiralinneren angegossen wird. Das so erhaltene Implantat weist eine Spiralform mit stark formschlüssigen Windungen auf.

Die erfindungsgemäße Vorrichtung zum Einbringen des Implantates kann im wesentlichen ein Rohr mit rechteckigem Querschnitt aufweisen in dem der elastische Körper eingezogen werden kann, und zwar vorzugsweise so weit, dass das Mittelstück noch aus dem distalen Ende des Rohres ragt. Der Querschnitt des Rohres entspricht im wesentlichen dem Querschnitt des elastischen Körpers. Der Vorschub des Körpers kann von Hand erfolgen. Erfindungsgemäß sind jedoch unter entsprechender Querschnittsvergrösserung des Rohres Vorschubmittel in Form eines Stössels vorgesehen, welche z.B. über einen Hebelmechanismus aktiviert werden können. Der Stössel weist an seinem distalen, in Wirkverbindung mit dem elastischen Körper oder der Versteifung stehenden Ende Mittel auf, die einen Vorschub des Körpers erleichtern. Die Mittel können darin bestehen, die Oberfläche aufzurauhen, oder aber auch in zahnartigen Fortsätzen mit Vorzugsorientierung in distaler Richtung. Dadurch wird erreicht, dass die Fortsätze bei Vorschub des Stössels in den elastischen Körper oder in die Versteifung eingreifen und ihn/sie mit sich schieben, während sie bei Rückzug des Stössels über die Oberfläche nach hinten gleiten. Dadurch ist ein kontrollierter Vorschub des elastischen Körpers möglich. Von zusätzlichem Vorteil ist es, das Rohr an seinem distalen Ende mit einer einseitigen Querschnittsverengung auszugestalten, welche an ihrem schmalen Ende nur noch den elastischen Körper aufnehmen kann. Dies bewirkt, dass der Stössel beim Vorschieben gleichzeitig nach unten gedrückt wird und so den Kraftschluss zwischen Stössel und Körper verstärkt.

Das Rohr der Einbringvorrichtung kann in vorteilhafter Weise auch gekrümmt sein, da das Einbringen des Implantates in den Zwischenwirbelraum vorzugsweise von seitlich dorsal erfolgt. Die Krümmung kann sowohl nach oben oder unten, sowie wahlweise nach links oder rechts ausgeführt sein, je nachdem von welcher Seite der Zugang erfolgen soll. Dabei kann die Einbringvorrichtung auch vorne keilförmig auslaufen, um benachbarte Wirbelkörper nach entstandenem Abstandsverlust wieder ausreichend mit dem keilförmigen Teil zu distanzieren.

Nachdem die Zwischenwirbelprothese implantiert worden ist, wird die Einführvorrichtung entlang der Versteifung, welche wenigstens teilweise ausserhalb des Bandscheibenbereichs verbleibt, zurückgezogen und ein Abtrenninstrument aufgeschoben. Dieses Instrument weist vorzugsweise einen Wirkmechanismus ähnlich einer Zange auf, umfassend zwei Griffteile, die über ein Gelenk miteinander in Verbindung stehen. Eine Backe des Schneidteiles ist hohl ausgebildet, sodass sie die Versteifung aufnehmen kann. Die andere Backe weist eine Schneide auf, die im wesentlichen quer zur Längsachse der Zange steht. Die Länge der Backen sowie die Anordnung der Schneide in distaler Richtung werden vorzugsweise so gewählt, dass einerseits ein Schnitt im Zwischenwirbelbereich möglich ist, andererseits noch die notwendigen Abtrennkräfte sicher aufgebracht werden können. Dabei ist der elastische Körper so umschlossen, dass beim Abtrennen des nicht benötigten Spiralenendes keine fremden Gewebeteile mitgeschnitten werden. Natürlich können auch Zangen Verwendung finden, deren Griffteile in einem Winkel zur Längsachse stehen.

In einer besonders bevorzugten Ausführung kann die Einbringvorrichtung auch ein Schneidinstrument beinhalten. Dies ist z.B. dadurch realisierbar, dass ein weiterer Stössel an seinem distalen Ende mit einer Schneidklinge versehen ist, die quer zur Längsachse der Vorrichtung verläuft. Die Schneidwirkung wird durch die distale Querschnittsverengung des Rohres, ähnlich wie beim Vorschub, erzeugt.

Das Implantat hat den Vorteil, möglichst einfach im Aufbau zu sein und eine Stützwirkung gleichmässig über einen Grossteil seiner Fläche zu sichern. Das Implantat hat ferner den Vorteil nicht durch zusätzliche Mittel in Spiralform fixiert werden zu müssen, sondern kann mit vergleichsweise einfachen und sicher zu bedienenden Instrumenten eingebracht werden.

Im folgenden wird die Erfindung anhand eines besonders bevorzugten Ausführungsbeispieles im Zusammenhang mit den Zeichnungen näher erläutert.
- Fig. 1: zeigt schematisch und stark vergrössert den elastischen Körper in Form einer Spirale;
- Fig. 2: zeigt einen Querschnitt durch die Spirale nach Fig. 1;
- Fig. 3: zeigt in schematischer Darstellung eine Einführvorrichtung;
- Fig. 4: zeigt in schematischer Darstellung eine Abtrennvorrichtung und
- Fig. 5: zeigt schematisch eine Draufsicht auf die Abtrennvorrichtung gemäss Fig. 4.

Die Figuren handeln von einem Implantat, insbesondere einer Zwischenwirbelprothese, welches aus einem länglichen elastischen Körper besteht, der formelastisch ist und im kräftefreien Zustand die Form einer Spirale S annimmt. Die Spirale lässt sich durch Rückwärtsaufziehen in ein Einführinstrument einziehen, welches im Einführbereich nur unwesentlich grösser als der Querschnitt des länglichen elastischen Körpers ist, um durch eine kleine Oeffnung im anulus fibrosus den Innenraum einer Bandscheibe zu erreichen und die sich selbst aufwickelnde Spirale einzuschieben und abzutrennen, wenn der Innenraum gefüllt ist. Dies hat den Vorteil, dass unterschiedlich grosse Innenräume mit der gleichen Spirale gefüllt werden können.

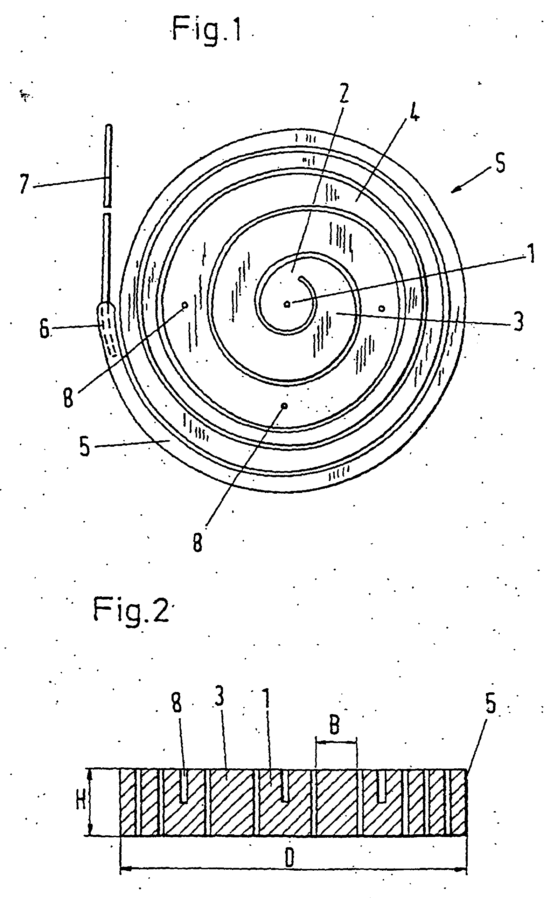

Fig. 1 zeigt das spiralförmige Implantat mit einem zentralen Mittelstück 1, das im wesentlichen die Form eines Zylinders aufweist. Daran schliesst seitlich ein dünnerer Bereich 2, der eine starke Abwinkelung und somit einen formschlüssigen Übergang zur daran anschliessenden Spiralwindung 3, im wesentlichen ohne Zwischenräume, ermöglicht.

Für den Fall, bei dem das Mittelstück entfällt empfiehlt es sich das innere Ende der Spiralwindung so auszubilden, dass bei Belastung keine Spannungspitzen auftreten können. Dies kann beispielsweise durch ein abgerundetes Ende erreicht werden.

Die an den Übergangsbereich anschliessenden Spiralwindungen sind formschlüssig und weisen eine konstante Breite im Bereich von 2 bis 5 mm auf, sodass ein Verkanten unter der zu erwartenden Belastung weitestgehend auszuschliessen ist. Das Implantat umfasst vorzugsweise 2-4 verkantungssichere Windungen auf. Daran schliesst ein Übergangsbereich 4 an, in dem die Breite des elastischen Körpers soweit reduziert wird, dass etwa im Bereich 5 von Fig. 1 ein leichtes Abtrennen möglich wird. Durch die Anzahl der Windungen wird die Spirale intra-operativ auf die gewünschte Grösse eingestellt. Die Stützwirkung bezüglich Kippen ist in den äusseren schmalen Windungen als solche vermindert, jedoch in Verbindung mit den formstabileren inneren Windungen ausreichend gross.

Die Dicke des elastischen Körpers, der die Spirale bildet, kann aber auch stetig von innen her abnehmen, sodass kein besonders ausgebildeter Übergangsbereich notwendig ist. In Fig. 2 ist die Spirale im Querschnitt dargestellt. Sie hat einen Durchmesser im Bereich von 20 bis 30 mm, vorzugsweise im Bereich von 23 bis 25,5 mm. Die Höhe beträgt vorzugsweise 5-10 mm. Die Höhe kann auch in zentraler Richtung kontinuierlich zunehmen und im Zentrum maximal 10 mm annehmen. Die Höhe kann auch dem individuellen Verlauf der Wirbelflächen angepasst sein, was insbesondere bei Wirbelkörperdeformationen von Interesse sein kann.

An das freie Ende der Spirale 6 ist eine bandförmige Versteifung 7 fixiert, welche über das freie Ende hinausragt und als Verlängerung beim Hantieren der Spirale dienlich ist. Der Übergang soll möglichst glatt sein. Das Band soll in der Lage sein Schubkräfte zu übertragen und wird aus Biokompatibilitäts- und Sterilisationsgründen vorzugsweise aus Titan gefertigt. Seine Länge beträgt praktischer Weise etwa 20 cm und ermöglicht eine Führung des Abtrenninstrumentes bis zur Abtrennstelle an der Spirale.

Der elastische Körper besteht vorzugsweise aus Kunststoff, etwa Polyurethan, wobei im kräftefreien Zustand die Spiralwindungen weitestgehend förmschlüssig zu liegen kommen. Die elastischen Eigenschaften des Materials müssen so sein, dass auch nach einem Ausrollen, wieder die Spiralform eingenommen wird. Die Deformationseigenschaften des Materials sollen denen der Bandscheibe weitestgehend ähnlich sein und vorteilhafter Weise etwa im Bereich der Shore-Härte von 70A bis 90A, vorzugsweise um 80A, liegen.

Der elastische Körper kann zu seiner Herstellung aus dem Werkstoff ausgeschnitten werden, was insbesondere mit computergesteuerten Lasern möglich ist. Schneidvorgänge, die das Material thermisch nicht belasten sind bevorzugt, da so toxische Nebenprodukte vermieden werden und auch auf die elastischen Eigenschaften des Materials kein Einfluss genommen wird. Es muss nicht besonders erwähnt werden, dass das Material als solches sterilisierbar sein soll.

Um die Lage des Implantates intra- und post-operativ kontrollieren zu können, empfiehlt sich das Vorsehen von röntgen-positiven Markierungen in Form von Punkten 8 oder eines in den Abbildungen nicht dargestellten Fadens auf bzw. entlang und innerhalb des elastischen Körpers. Als Markermaterial kann Barium, Tantal oder Ähnliches Verwendung finden.

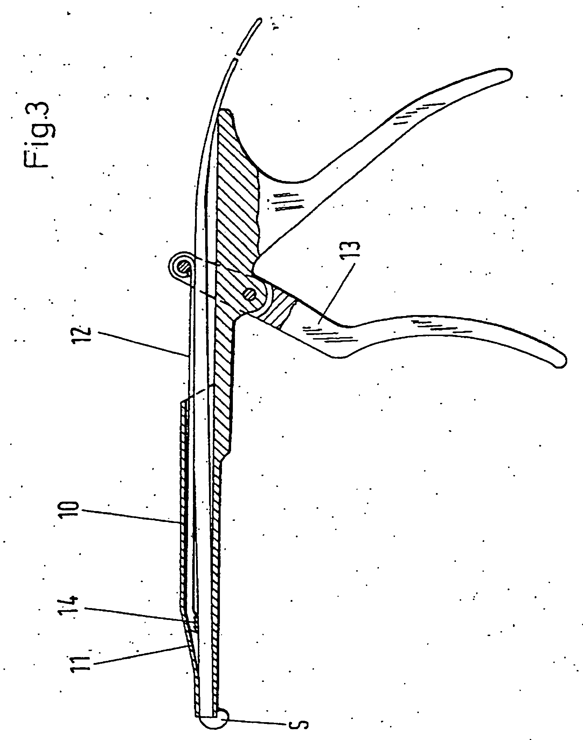

Die Vorrichtung zum Einbringen des elastischen Körpers ist in Fig. 3 näher dargestellt. Sie umfasst ein Vierkantrohr 10 zur Aufnahme der wenigstens soweit ausgerollten Spirale, dass ihr noch vorstehender Teil S problemlos durch die operativ geschaffene Öffnung in den Zwischenwirbelbereich eingebracht werden kann. Das Hohlrohr 10 weist vorzugsweise eine einseitige Querschnittsverengung 11 am distalen Ende auf, welche konisch oder schnabelartig ausgebildet ist. Das Rohr kann auch eine Krümmung aufweisen, die den Zugang zum Zwischenwirbelbereich von seitlich dorsal erleichtert und auf die Händigkeit des Benützers abgestellt ist. Die Krümmung kann aber auch nach caudal oder cranial gerichtet sein, je nach Anforderung.

Der Vorschub des Körpers kann auch von Hand erfolgen. Hierbei sind Griffmittel um das Rohr abzustützen hilfreich. Sie können koaxial mit dem Rohr in Form einer Verdickung ausgebildet sein oder radial zur Rohrachse verlaufen. Vorzugsweise sind jedoch Vorschubmittel in Form eines elastischen Stössels 12 vorgesehen, welche z.B. über einen Hebelmechanismus 13 betrieben werden können. Der Stössel weist an seinem distalen, in Wirkverbindung mit dem elastischen Körper oder mit der Versteifung stehenden Ende Mittel 14 auf, die einen Vorschub des Körpers erleichtern. Die Mittel können darin bestehen, die Oberfläche aufzurauhen, oder aber auch in zahnartigen Fortsätzen mit Vorzugsorientierung in distaler Richtung. Dadurch wird erreicht, dass die Fortsätze bei Vorschub des Stössels in den elastischen Körper oder in die Versteifung eingreifen und ihn/sie mit sich schieben, während sie bei Rückzug des Stössels über die Oberfläche nach hinten gleiten. Dabei ist ein kontrollierter Vorschub des elastischen Körpers möglich. Die einseitige Querschnittsverengung 11 des Vierkantrohres 10 bewirkt, dass der Stössel beim Vorschieben gleichzeitig nach unten gedrückt wird und so den Kraftschluss zwischen Stössel und Körper verstärkt. Dadurch dass der Stössel sehr weit vorne an der abgewickelten Spirale angreift, wird diese praktisch durch das Hohlrohr gezogen, sodass sie sich nicht unter ihrer Eigenspannung selbst blockiert, wie es bei Schub von hinten leicht der Fall sein könnte.

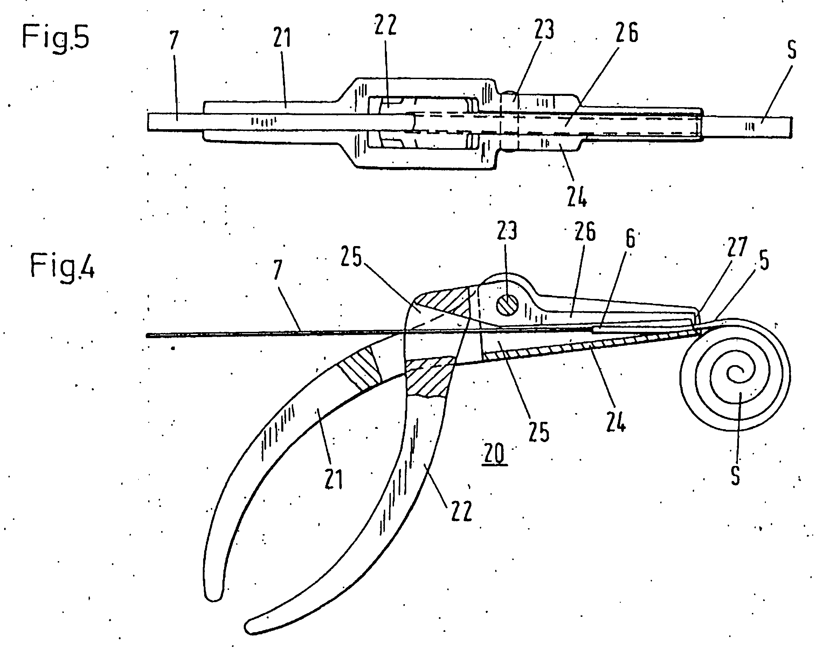

Fig. 4 und 5 zeigen eine Vorrichtung zum Abtrennen des überflüssigen Spiralenendes. Nachdem die Zwischenwirbelprothese implantiert worden ist, wird die Einführvorrichtung entlang der Versteifung 7, welche ausserhalb des Bandscheibenbereichs verbleibt, zurückgezogen und das Abtrenninstrument 20 aufgeschoben. Dieses Instrument weist vorzugsweise einen Wirkmechanismus ähnlich einer Zange auf, umfassend zwei Griffteile 21,22, die über ein Gelenk 23 miteinander in Verbindung stehen. Eine Backe 24 ist in Längsrichtung mit einer Ausnehmung 25 versehen, in die die Versteifung 7 und/oder der elastische Körper 5,6 aufgenommen werden kann. Die andere Backe 26 weist eine Schneide 27 auf, die im wesentlichen quer zur Ausnehmung 25 beweglich ist. Die Länge der Backen sowie die Anordnung der Schneide in distaler Richtung wird vorzugsweise so gewählt, dass einerseits ein Schnitt innerhalb des Zwischenwirbelbereiches möglich ist, andererseits noch die notwendigen Abtrennkräfte sicher aufgebracht werden können. Die Schneidklinge selbst kann als auswechselbare Klinge gestaltet sein. Natürlich können auch Zangen Verwendung finden, deren Griffteile in einem Winkel zur Längsachse stehen.

Nachdem der elastische Körper auf die gewünschte Länge gekürzt worden ist, wird die Abtrennvorrichtung zusammen mit der Versteifung 7 zurückgezogen. Das Implantat nimmt aufgrund seiner formstabilen Eigenschaften die Spiralform an. Das Implantat behält auch unter Belastung seine Spiralform bei, ohne dass eine besondere Fixierung des freien Endes notwendig wäre.

Das zuvor beschriebene Verfahren zum Einbringen eines spiralförmigen Implantates in einen Hohlraum, kommt vorteilhafter Weise auch zu Lehr-, Übungs-, und Demonstrationszwecken zur Anwendung.

## Patentansprüche

1. Vorrichtung zum Einbringen eines Implantats, insbesondere einer Zwischenwirbelprothese, bestehend aus einem länglichen, elastischen Körper, welcher formelastisch ist und im kräftefreien Zustand die Form einer Spirale (S) annimmt, wobei in Form eines Aufnahmerohres (10) ausgebildete Mittel zur Aufnahme wenigstens eines Teiles der Spirale (S) und Mittel (12,13) zum dosierten Vorschieben der Spirale vorgesehen sind
**dadurch gekennzeichnet ,**
**dass** unter entsprechender Querschnittsvergrößerung des Aufnahmerohres (10) für den Körper (S) Vorschubmittel in Form eines Stössels (12) vorgesehen sind, der an seinem distalen, in Wirkverbindung mit dem Körper (S) stehenden Ende Mittel (14) aufweist, die einen Vorschub des Körpers (S) erleichtern.

2. Vorrichtung nach Anspruch 1, **dadurch gekennzeichnet, dass** die Mittel zur Aufnahme ein hohles Rohr (10) mit rechteckigem Querschnitt umfassen, dessen distales Ende eine Abschrägung (11) aufweist, um ein Einführen in den Zwischenwirbelbereich zu erleichtern, wobei die Vorschubmittel in Form eines länglichen, vorzugsweise biegeelastischen Stössels (12) innerhalb des hohlen Rohres vorgesehen sind und der Stössel an seinem distalen Ende zahnartige Fortsätze (14) aufweist, die durch die distale Abschrägung des Rohres auf den elastischen Körper hin in Wirkverbindung gebracht werden, so dass ein Vorschub der Stange eine Mitnahme des elastischen Körpers bewirkt.

3. Vorrichtung nach Anspruch 2, **dadurch gekennzeichnet, dass** die Zähne (14) in distaler Richtung hin gerichtet sind, so dass bei Rückzug des Stössels ein Gleiten auf der Unterlage erfolgt, bei Vorschub jedoch ein Greifen.

4. Vorrichtung nach Anspruch 2, **dadurch gekennzeichnet, dass** die Mittel zur Aufnahme (10) an ihrem distalen Ende eine Krümmung aufweisen.

5. Vorrichtung nach Anspruch 1, **dadurch gekennzeichnet, dass** im distalen Ende eine Abschneidvorrichtung zum Abschneiden der elastischen Spirale vorgesehen ist.

## Claims

1. An apparatus for the introduction of an implant, in particular of an intervertebral prosthesis, consisting of an elongated elastic body which is elastic in shape and adopts the shape of a spiral (S) in the force-free state, wherein means made in the form of a reception tube (10) are provided for the reception of at least one part of the spiral (S) and means (12, 13) are provided for the metered advancing of the spiral,
**characterised in that**
advancing means in the form of a pusher (12) are provided with a corresponding cross-sectional increase of the reception tube (10) for the body (S), said pusher having means (14) at its distal end in operative communication with the body (S) which facilitate an advancing of the body (S).

2. An apparatus in accordance with claim 1, **characterised in that** the means for the reception comprise a tube (10) having a rectangular cross-section whose distal end has a chamfer (11) to facilitate an introduction into the intervertebral area, with the advancing means being provided in the form of an elongate, preferably flectionally elastic pusher (12) inside the hollow tube and the pusher having tooth-like prolongations (14) at its distal end which are brought into operative communication towards the elastic body by the distal chamfer of the tube so that an advancing of the rod effects a taking along of the elastic body.

3. An apparatus in accordance with claim 2, **characterised in that** the teeth (14) are aligned in the distal direction such that a sliding takes place on the support on a withdrawal of the pusher, but a gripping on the advancing.

4. An apparatus in accordance with claim 2, **characterised in that** the means for the reception (10) have a curvature at their distal end.

5. An apparatus in accordance with claim 1, **characterised in that** a cutting device is provided in the distal end for the cutting off of the elastic spiral.

## Revendications

1. Dispositif pour placer un implant, en particulier une prothèse intervertébrale, constitué par un corps allongé élastique, qui est élastique quant à sa forme et qui prend, en l'absence de contraintes, la forme d'une spirale (S), des moyens réalisés sous la forme d'un tube de réception (10) étant prévus pour la réception d'au moins une partie de la spirale (S) et des moyens (12, 13) étant prévus pour faire avancer de manière dosée la spirale, **caractérisé en ce qu'**en agrandissant de manière correspondante la section transversale du tube de réception (10) pour le corps (S), des moyens d'avancement sont prévus sous forme d'un poussoir (12) qui présente à son extrémité distale se trouvant en liaison active avec le corps (S), des moyens (14) qui facilitent un avancement du corps (S).

2. Dispositif selon la revendication 1, **caractérisé en ce que** les moyens de réception comprennent un tube (10) creux avec section transversale rectangulaire dont l'extrémité distale présente un chanfrein (11) pour faciliter une introduction dans la région intervertébrale, les moyens d'avancement sous forme d'un poussoir (12) allongé, de préférence élastique à la flexion sont prévus à l'intérieur du tube creux et le poussoir présente à son extrémité distale des prolongements (14) en forme de dents qui sont amenés en liaison active vers le corps élastique par le chanfrein distal du tube, de sorte qu'un avancement de la tige suscite un entraînement du corps élastique.

3. Dispositif selon la revendication 2, **caractérisé en ce que** les dents (14) sont orientées en direction distale, de telle sorte que lors du retrait du poussoir se produit un glissement sur la base, mais toutefois un engagement lors d'un avancement.

4. Dispositif selon la revendication 2, **caractérisé en ce que** les moyens de réception (10) présentent une courbure à leur extrémité distale.

5. Dispositif selon la revendication 1, **caractérisé en ce que** dans l'extrémité distale est prévu un dispositif de coupe pour couper la spirale élastique.
